# EUROPEAN PATENT APPLICATION

(11) **EP 1 277 479 A2**
(43) Date of publication of application: **22.01.2003**
(21) Application number: 01202319.8
(22) Date of filing: 18.06.2001
(51) Int. Cl.: A61K 47/12, A61K 9/16

(54) **Alkali or alkaline earth metal benzoate particles**

(71) Applicant: DSM N.V., 6411 TE Heerlen (NL)
(72) Inventor: Schaafsma, Stefan, 6132 SM Sittard (NL)
(74) Representative: Jacobs, Monique S.N.

(57) **Abstract**

The invention relates to alkali or alkaline earth metal benzoate particles having a mean particle diameter (d₅₀) smaller than 500 µm and a flow value according to the vessel method of ≤ 4 and to a fluidized-bed spray granulation process for the production of alkali or alkaline earth metal benzoate particles.

The invention further relates to a process for the production of compacts comprising alkali or alkaline earth metal benzoate by direct compression of alkali or alkaline earth metal benzoate particles with a bulk density higher than 400 kg/m³ and an angle of repose smaller than 40 °.

The invention also relates to the use of alkali or alkaline earth metal benzoate as a carrier and to the use of alkali or alkaline earth metal benzoate particles according to the invention in a capsule or a sachet.

## Description

The invention relates to alkali or alkaline earth metal benzoate particles and to a process for the production of alkali or alkaline earth metal benzoate particles. The invention also relates to a process for the production of compacts comprising alkali or alkaline earth metal benzoate, to the use of alkali or alkaline earth metal benzoate particles in a capsule or a sachet and to the use of alkali or alkaline earth metal benzoate as a carrier.

The invention relates to alkali or alkaline earth metal benzoate particles with a mean particle diameter (d₅₀) smaller than 500 µm and a flow value according to the vessel method ≤ 4. It was surprisingly found that alkali or alkaline earth metal benzoate particles, particularly alkali or alkaline earth metal bezoate particles with such small particlesize, feature excellent flow behaviour compacting properties so that they are suitable for many applications, for instance compacting. Alkali or alkaline earth metal benzoate particles can also be used in other demanding applications, for instance in capsules and sachets and as a carrier.

The d₅₀ of the alkali or alkaline earth metal benzoate particles is smaller than 500 µm, preferably smaller than 250 µm. Herein d₅₀ is defined as the mean mass-based particle diameter. The small particle size of the alkali or alkaline earth metal benzoate particles results in a relatively large surface area compared to larger particles, which makes the particles particularly suitable as e.g. a carrier.

The flow value of the alkali or alkaline earth metal benzoate particles, measured according to the vessel method, is ≤ 4, preferably ≤ 3, more preferably ≤ 2 and in particular 1. Alkali or alkaline earth metal benzoate particles with a low flow value have a reduced tendency to clogging, while preparation of an accurate dosage of the particles, which is particularly important for pharmaceutical applications, is facilitated.

The alkali or alkaline earth metal benzoate particles according to the invention preferably have a bulk density higher than 400 kg/m³, more preferably the bulk density higher than 500 kg/m³, in particular higher than 550 kg/m³ and more in particular higher than 600 kg/m³. Herein the bulk density is measured according to ASTM D 1895-89 (method A). It is surprising that alkali or alkaline earth metal benzoate particles which such a high bulk value could be produced.

A high bulk density is favoured from a packaging point of view. Stacks of bags filled with product having a high bulk density are more stable and less inclined to sag and/or collapse and are thereby consequently saver. Furthermore, a high bulk density reduces transport costs by transporting the same amount of material in less volume.

The angle of repose of the alkali or alkaline earth metal benzoate particles according to the invention, measured according to DIN/ISO 4324, is preferably smaller than 40 °, more preferably smaller than 35 °, in particular smaller than 30 °.

The crushability of the of the alkali or alkaline earth metal benzoate particles is preferably low to avoid the formation of dust, which could cause segregation of the particles and is undesirable for hygiene and safety reasons. When the particles are used as a carrier for an entity a low crushability further contributes to the homogeneity of the entity on the carrier, which facilitates accurate dosing. The crushability of the particles according to the invention is preferably lower than 20 wt%, more preferably lower than 10 wt%, in particular lower than 5 wt%.

For many applications alkali or alkaline earth metal benzoate particles are dissolved in a liquid. Sodium benzoate, for instance, is used as a preservative in soft drinks and in injection solutions (IV and IM injections). A fast dissolution of the alkali or alkaline earth metal benzoate particles is favoured as it saves preparation time of the application. The alkali or alkaline earth metal benzoate particles according to the invention have been found to disintegrate and dissolve quickly.

Alkali or alkaline earth metal benzoate particles are particularly suitable for the use in a formulation for the production of compacts, for example tablets, pellets and briquettes, by direct compression. It has surprisingly been found that compacts made by direct compression of formulations comprising of alkali or alkaline earth metal benzoate particles have a low friability, a high hardness, a high dilution potential, and a good disintegration time with a model drug.

The alkali or alkaline earth metal benzoate particles according to the invention can for instance be prepared by fluidized-bed spray granulation. The invention also relates to such a process for the production of alkali or alkaline earth metal benzoate particles

A fluidized-bed spray granulation process combines the steps of crystallizing and shaping a solid in one process and is described by S. Heinrich in
"Modellierung des Warme- und Stoffübergangs sowie der Partikelpopulationen bei der Wirbelschicht Sprühgranulation", Fortschritt-Berichte VDI, nr 675, 2001. In the fluidized-bed spray granulation process according to the invention a liquid, preferably a clear liquid, more preferably water, containing alkali or alkaline earth metal benzoate, is sprayed upon an (air-)fluidized-bed containing alkali or alkaline earth metal benzoate particles. Optionally, seeds can be added to the process.

While the liquid evaporates, the alkali or alkaline earth metal benzoate crystallizes on the surface and in the pores of the alkali or alkaline earth metal benzoate particles. As such the particles grow by layering. The size of the alkali or alkaline earth metal benzoate particles can be regulated by the residence time in the fluidized bed and the droplet size distribution of the alkali or alkaline earth metal benzoate solution sprayed by the nozzles. As a side effect, dust consisting of very small particles of alkali or alkaline earth metal benzoate is formed due to spray-drying of small droplets in their trajectory to the alkali or alkaline earth metal benzoate particles. The fluidization (air) velocity controls the number and size of the alkali or alkaline earth metal benzoate particles. The fluidization (air) velocity can be chosen such that the desired amount of dust is present in the product. If desired, the product can be sieved. The optimum process configuration for a specific application can easily be established by the skilled person.

Fluidized-bed spray granulation can be used to produce alkali or alkaline earth metal benzoate particles with a low crushability, a relatively low dust content and a low angle of repose, which are suitable for use in for instance compacts, capsules and sachets and for use as a carrier.

Preferably, the alkali or alkaline earth metal benzoate particles produced by fluidized-bed spray granulation have a d₅₀ lower than 500 µm, more preferably lower than 250 µm. Surprisingly these particles of alkali or alkaline earth metal benzoate combine a small particle size with excellent flow properties.

The invention also relates to a process for the production of compacts comprising alkali or alkaline earth metal benzoate. According to the invention the compacts are produced by direct compression of a formulation comprising alkali or alkaline earth metal benzoate particles with a bulk density higher than 400 kg/m³ and an angle of repose lower than 40 °.

In the framework of the invention direct compression involves dry blending of the alkali or alkaline earth metal benzoate particles with the other ingredients of the desired formulation, directly followed by compression. As direct
compression does not require the wet or dry granulation step it is a favoured technique, as described in for example Ullmann's Encyclopedia of Industrial Chemistry, Sixth Edition, 1999. High-speed (rotary) presses can nowadays produce more than 10.000 compacts per minute. To be used in such processes, the compression properties of the alkali or alkaline earth metal benzoate particles must be sufficient to ensure compact quality, preferably even at high compacting speeds, preferably higher than 50 mm/sec, more preferably 100 mm/sec, in particular higher than 150 mm/sec, more in particular higher than 300 mm/sec, which are preferred for economical reasons. The compaction pressure used in the compacting process is dependent on for instance the size and the shape of the die and the product properties of the other components in the formulation of the compact, and is preferably higher than 7 MPa, more preferably higher than 75 MPa, in particular higher than 150 MPa, more in particular higher than 225 MPa. It is very surprising that formulations can be provided containing alkali or alkaline earth metal benzoate particles that can be subjection to direct compression to yield compacts, which feature high compacting process stability, high compact to compact consistency, high hardness, favourable disintegration time and low friability.

The bulk density of the alkali or alkaline earth metal benzoate particles used in formulations for the production of compacts by direct compression is higher than 400 kg/m³, preferably higher than 500 kg/m³, more preferably higher than 550 kg/m³, in particular higher than 600 kg/m³. A high bulk density results in a low bulk volume and consequently a uniform filling of the die, which contributes to the compact quality.

The angle of repose of the alkali or alkaline earth metal benzoate particles used in formulations for the production of compacts by direct compression is preferably smaller than 40 °, more preferably smaller than 35°, in particular smaller than 30 °. Particles featuring a small angle of repose show a reduced tendency to clogging, while preparation of an accurate dosage of the particles, which is particularly important for pharmaceutical applications, is facilitated.

The d₅₀ of the alkali or alkaline earth metal benzoate particles used in formulations for direct compression of compacts is presently as a rule lower than 2 mm. The d₅₀ of the alkali or alkaline earth metal bezoate particles preferably used in direct compression processes depends on the size of the compacts to be produced. The particle size is chosen in function of for instance the other components in the formulation such that no seggregation occurs between the particles. Typically, the particle diameter is at least 10 times, preferably at least 20 times smaller than the smallest diameter of the die.

Consequently, for medium-sized compacts, i.e. compacts with a diameter between 5 and 10 mm, preferably particles with a d₅₀ less than 1 mm, more preferably less than 500 µm are used. For small compacts, i.e. compacts with a diameter less than 5 mm, preferably particles with a d₅₀ less than 500 µm, more preferably less than 250 µm are used.

The crushability of the alkali or alkaline earth metal benzoate particles used in formulations for the production of compacts by direct compression is preferably lower than 20 wt%, more preferably lower than 10 wt%, in particular lower than 5 wt%.

Preferably the alkali or alkaline earth metal benzoate particles used for the production of compacts by direct compression are sodium benzoate particles. Sodium benzoate is a well-known antimicrobal preservative in cosmetics, foods and pharmaceuticals. Sodium benzoate, like other benzoate salts, is tolerated in large quantities, which is demonstrated by the fact that e.g. 6 g of sodium benzoate in 200 mL of water is administered orally in a liver function test. Ingested sodium benzoate is conjugated with glycine in the liver to yield hippuric acid, which is excreted in the urine. Sodium benzoate is GRAS listed, accepted as a food additive in Europe and included in the FDA Inactive Ingredients Guide (dental preparations, IM an IV injections, oral capsules, solutions and compacts, rectal and topical preparations) (see for example "Handbook of Pharmaceutical Excipients", Ed. A.H. Kibbe, American Pharmaceutical Association, Washington DC USA, p. 471-473, 2000).

Another aspect of the invention concerns the use of alkali or alkaline earth metal benzoate particles according to the invention in capsules and sachets. The high flow value and the high bulk density of the alkali or alkaline earth metal benzoate particles according to the invention facilitate accurate dosing, while the low dust content, the low crushability and the low friability contribute to the product uniformity and prevent seggregation in the formulation.

Alkali or alkaline earth metal benzoate particles according to the invention are very suitable for use in capsules and sachets as a glident, lubricant and/or diluent aid, potentially improving blend composition homogeneity.

Particularly suitable for the use in capsules and sachets are alkali or alkaline earth metal benzoate particles which are produced by fluidized-bed spray granulation.

Yet another aspect of the alkali or alkaline earth particles according to the invention concerns the use of alkali or alkaline earth metal benzoate as a carrier, in which the carrier is used as a support for an entity. In the scope of the invention the support is used for supporting the entity in order to obtain a material with enhanced properties relative to the entity. Examples of entities are pharmaceuticals, food and home and personal care products. Carriers can be used for example in an inhaler dosage formulation.

The d₅₀ of the alkali or alkaline earth metal benzoate particles used as a carrier is preferably lower than 1 mm, more preferably lower than 500 µm, in particular lower than 250 µm. The smaller the particle size of the particles, the higher the surface area per mass of the particles, which contributes to the loading capacity of the particle for the entity.

The bulk density of the alkali or alkaline earth metal benzoate particles used as a carrier is preferably higher than 500 kg/m³, more preferably higher than 550 kg/m³, and in particular higher than 600 kg/m³. A high bulk density is favoured from a packaging point of view. Stacks of bags filled with product having a high bulk density are more stable and less inclined to sag and/or collapse and are consequently saver. Furthermore, a high bulk density reduces transport costs by transporting the same amount of material in less volume.

The angle of repose of the alkali or alkaline earth metal benzoate particles used as a carrier is preferably smaller than 40 °, more preferably smaller than 35 °, in particular smaller than 30 °.

The crushability of the alkali or alkaline earth metal benzoate particles used as a carrier is preferably low to avoid the formation of dust, which could cause segregation of the particles and is undesirable for hygiene and safety reasons. A low crushability further contributes to the homogeneity of the entity on the carrier, which facilitates accurate dosing. The crushability of the alkali or alkaline earth metal benzoate particles used as a carrier is preferably lower than 20 wt%, more preferably lower than 10 wt%, in particular lower than 5 wt%.

The invention will now be explained in more detail with reference to the following examples and comparative examples, without however being limited thereto.

### Experimental methods

### Testing of the fluidized-bed spray granulated particles

Particle size and particle size distribution were measured using laser diffraction (Sympatec, Germany), except the particle size and particle size distribution of samples E and F, which were determined by sieve analysis according to DIN 66165.

The flow value was determined by the vessel method according to Klein in K. Klein; Seiten, Öle, Fette, Wachse, 94, 849 (1968), which involves a series of vessels, each with an opening at the bottom of the vessel. The openings in the vessels have different sizes. Vessel 1 has the smallest opening of 2.5 mm. Vessel 5 has the largest opening of 18 mm. The openings of vessels 2 to 4 are 5, 8 and 12 mm respectively. The flow value of particles corresponds to the number of the vessel with the smallest opening the particles can flow through. The particles with the lowest flow value have the best flow properties.

The bulk density was measured according to ASTM D 1895-89 (method A).

The crushability is a measure of the hardness of particles and was determined according to the so-called ball test. 50 g of particles and 36 steel balls (diameter 15 mm, weight 13.7 gram) were introduced into a sieve pan, placed on a shaking screen and shaken for 5 minutes. The sieve pan must perform a circular movement in the horizontal plane at a frequency of about 250 revolutions per minute and an amplitude (top to top) of 15 mm. For particles between 425 and 500 µm the mesh width of the shaking screen is preferably 250 µm. The weight percentage of the particles smaller than 250 µm relative to the amount of starting material (50 gram) is the crushability.

### Preparation of samples and method of compacting

Compacts comprising sodium benzoate were produced under standard conditions (hydraulic press, 500 mg compact weight, 13 mm tablet diameter). The compaction force was varied between 5 and 35 kN and the compression speed was 3 or 300 mm/s. The formulation with the pharmaceutical model compound Theophyllicum monohydricum (Genfarma b.v., Maarsen, sieved at 180 micrometer) used in the compacts was produced by mixing a dry blend of sodium benzoate particles and Theophyllicum monohydricum for 10 minutes in a Turbula mixer at 90 rpm. Some compacts were lubricated with 0.5 % magnesium stearate (Janssen Chimica). For this purpose magnesium stearate was mixed with the formulation in a Turbula mixer at 90 rpm for 2 minutes.

### Testing of compacts

A disintegration tester according to the European Pharmacopeia Ed III, without the use of disks, determined the disintegration of the compacts. An average of six compacts was used, unless otherwise noted.

The Roche Friabilator determined the friability of the compacts according to the European Pharmacopeia Ed III.

A Schleuniger diametrical crushing strength apparatus measured the crushing strength. An average of six compacts was used, unless otherwise noted.

The dissolution rate of Theophyllicum monohydricum compacts was determined with a USP XXIII paddle apparatus (Rhone-Poulenc, Paris, France) in a 0.005 M phosphate buffer at pH 6.8. The concentration of Theophyllicum monohydricum was measured spectrophotometrically at 268 nm in triplicate using an Ultrospec 4052 TDS apparatus (LKB, Zoetermeer, The Netherlands).

### Examples

### Example 1. Fluidized-bed spray granulation of sodium benzoate particles

The fluidized bed had a surface area of 0,2 m² and contained 3 two-fluid nozzles (Schlick-nozzles). The air velocity (superficial velocity through the bed at bed temperature) was about 1.3 - 1.4 m/s and the maximum air inlet temperature applied was 280 °C. The bed temperature was kept constant at 75 - 80 °C. The liquid sprayed in the bed was water containing 37% (w/w) sodium benzoate at a temperature of 80 °C. The amount of liquid sprayed on the bed was varied to keep the bed temperature constant, the maximum spray rate being 85 kg/hr. The bed content was about 15 kg of sodium benzoate particles. The residence time was 0,5 - 1 hour depending on the throughput. Cleaning of the internal filters (pleated filter cartridges) took place by pressure pulses. The granulation was performed continuously during 25 hours. External seeds were used to control the particle size in the first 10 hours of the granulation process.

The seeds with a d₅₀ of 200 µm consisted of milled and subsequently sieved sodium benzoate particles. After 13 hours of granulation the fluidized bed was
kept stable at an inlet temperature of 260 °C for 10 hours. The bulk density of the particles varied between 580 kg/m³ and 620 kg/m³ during this period. The d₅₀ varied between 350 and 500 µm during this period.

### Example 2. Properties of sodium benzoate particles according to the invention

Sodium benzoate particles according to Example 1 were sieved according to DIN/ISO 4610. Fractions of particles were collected by sieving between 425 and 75µm, 250 and 45µm, smaller than 180 µm, and smaller than 125µm, yielding sodium benzoate particles with a d₅₀ of 250, 197, 118 and 99 µm, respectively. The properties of the fractions are listed in Table 1.

**Table 1.**

| Properties of sodium benzoate particles according to the invention. | | | | | |
|---|---|---|---|---|---|
| Sample | d₅₀ (µm) | Flow value (-) | Angle of repose (°) | bulk density (kg/m³) | Crushability (wt%) |
| 1 | 250 | 1 | 29 | 610 | 0.7 |
| 2 | 197 | 1 | 32 | 611 | 0.7 |
| 3 | 118 | 1 | 27 | 639 | 0.7 |
| 4 | 99 | 1 | 27 | 656 | 0.7 |

### Comparative example A. Properties of known sodium benzoate particles

**Table 2.**

| Properties of known sodium benzoate particles | | | | | |
|---|---|---|---|---|---|
| Sample | d₅₀(µm) | Flow value (-) | Angle of repose (°) | bulk density (kg/m³) | Crushability (wt%) |
| A | 130 | >5 | 47 | 324 | n.a. |
| B | 235 | >5 | 44 | 284 | n.a. |
| C | 40 | 5 | 35 | 308 | n.a. |
| D | 169 | > 5 | 45 | 546 | n.a. |
| E | 668 | n.a. | 46 | 710 | 21.1 |
| F | 517 | n.a. | 39 | 541 | 28.4 |
| n.a. = not analysed | | | | | |
| A is drum-dried sodium benzoate (Fushimi). | | | | | |
| B is drum-dried sodium benzoate (Paniplus). | | | | | |
| C is spray-dried sodium benzoate (Falanx). | | | | | |
| D is drum-dried sodium benzoate (Kalama). | | | | | |
| E is drum dried sodium benzoate (DSM) | | | | | |
| F is drum dried sodium benzoate (DSM) | | | | | |

### Example 3. Compacting of sodium benzoate particles according to the invention

Sodium benzoate particles according to the invention (sample 1 and 2) were compacted using a tablet press. A sample obtained by fluidized-bed spray granulation after between 19 and 20 hours of granulation was used. The d₅₀ of this batch of particles was 444 µm. Sieved fractions of this batch were used for compression. Results are shown in Figures 1 and 2. The results show very high crushing strengths for the compacts. There is no significant difference between the compaction behaviour of compacts produced in low-speed (3 mm/s) and highspeed (300 mm/s) compaction processes. Both samples were lubricated. However, sodium benzoate has lubricating properties of its own and lubrication by another compound may not be necessary.

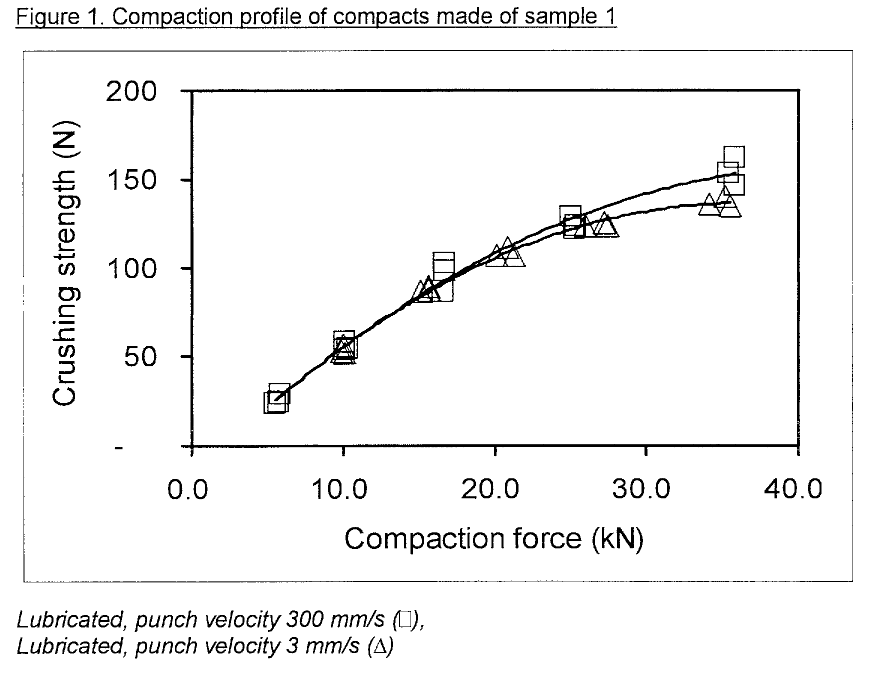

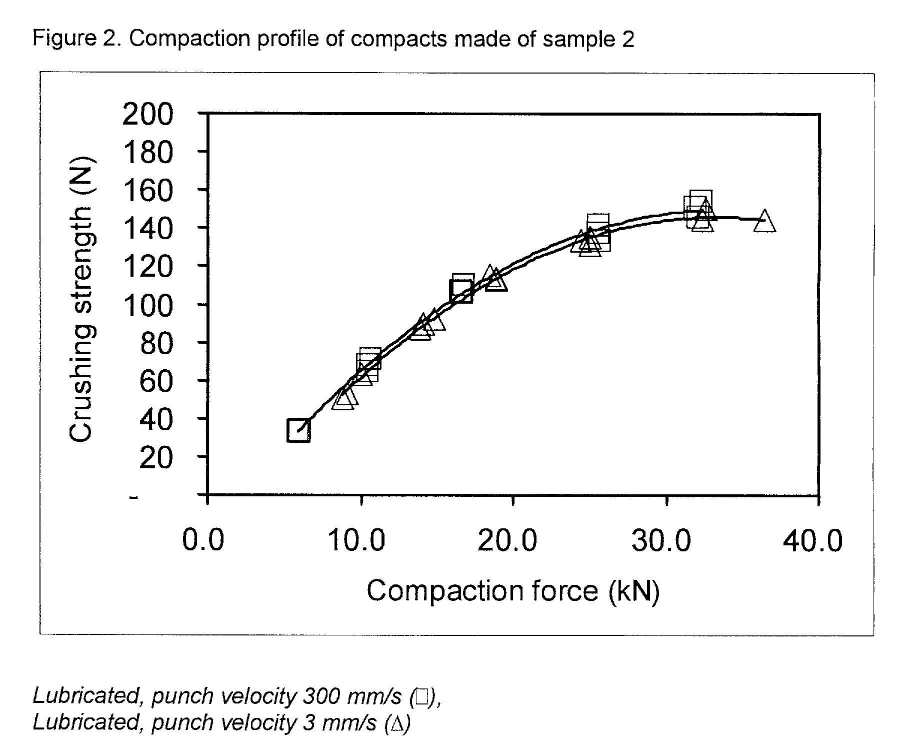

### Example 4. Crushing strength of compacts of sodium benzoate particles

### according to the invention

The crushing strength for compacts of samples 1-2, obtained by direct compression using a compression speed of 300 mm/s and a compression force of 20 kN, is give in Table 3. Magnesium stearate was used as a lubricant.

**Table 3.**

| Crushing strength for compacts of samples 1-2 and A-D. | |
|---|---|
| Sample | Crushing strength (N) |
| 1 | 110 |
| 2 | 120 |
| A | * |
| B | * |
| C | * |
| D | * |

| | |
|---|---|
| * could not be compressed | |

Table 3 shows that samples 1 and 2 could be compressed at a compression force of 20 kN without the occurrence of capping. A compaction force of 20 kN is a typical compaction force applied for compacts with a diameter of 13 mm for pharmaceutical applications.

### Comparative example B. Crushing strength of compacts of known sodium

### benzoate particles

The crushing strength for compacts of samples A-D, made by direct compression using a compression speed of 300 mm/s and a compression force of 20 kN, is give in Table 3. Magnesium stearate was used as a lubricant in all cases.
Table 3 shows that A-D could not be compressed at 20kN due to capping of the compacts.

### Example 5. Properties of compacts of pure sodium benzoate and of formulations

### of sodium benzoate and a model drug

Compacts of formulations of Theophyllicum monohydricum with sodium benzoate as filler and/or binder were made by direct compression using a compression speed of 300 mm/s and a compression force of 20 kN. Properties of the compacts are presented in Table 4.

**Table 4.**

| Sodium benzoate with Theophyllicum monohydricum | | | | |
|---|---|---|---|---|
| Sample | Compression force (kN) | Crushing strength (N) | Compact friability (%) | Disintegration time (s) |
| Sample 2 (not lubricated) | 20 | 167(17) | 0.71 | 227(6) |
| Sample 2 (lubricated) | 20 | 120(6) | 0.84 | 228(8) |
| Sample 2 with 10 wt% Theophyllicum monohydricum (lubricated) | 20 | 132 (4) | 0.69 | 334 (35) |
| Sample 2 with 40 wt% Theophyllicum monohydricum (lubricated) | 20 | 143(11) | 0.61 | 802(144) |

The crushing strength of the compacts is sufficient for application in pharmaceuticals. The friability is low. The disintegration of the compacts is fast; for the compacts containing theophyllicum monohydricum 80% of the drug was dissolved within 20 minutes. The properties of the compact with 40 wt% of theophyllicum monohydricum show that the dilution potential, i.e. the amount of drug which can be present without significantly deteriorating the compact properties, is very high.

The examples show that the sodium benzoate particles according to the invention have excellent compression properties and can be used in direct compression. The entity used together with sodium benzoate in a formulation for compacts may be blended with or sprayed on the sodium benzoate particles before compression.

## Claims

1. Alkali or alkaline earth metal benzoate particles having a mean particle diameter (d₅₀) smaller than 500 µm and a flow value according to the vessel method of ≤ 4.

2. Alkali or alkaline earth metal benzoate particles according to claim 1, **characterized in that** the crushability according to the ball test is lower than 20 wt%.

3. Alkali or alkaline earth metal benzoate particles according to claim 1 or 2 obtainable by fluidized-bed spray granulation.

4. Alkali or alkaline earth metal benzoate particles according to any one of claims 1-3, **characterized in that** the alkali or alkaline earth metal benzoate particles are sodium benzoate particles.

5. Process for the production of alkali or alkaline earth metal benzoate particles according to any one of claims 1-4, **characterized in that** the alkali or alkaline earth metal benzoate particles are produced by fluidized-bed spray granulation.

6. Process for the production of compacts comprising alkali or alkaline earth metal benzoate by direct compression of alkali or alkaline earth metal benzoate particles with a bulk density higher than 400 kg/m³ and an angle of repose smaller than 40 °.

7. Process according to claim 6, **characterized in that** the alkali or alkaline earth metal benzoate particles are produced by fluidized-bed spray granulation.

8. Process according to claim 6 or 7, **characterized in that** the alkali or alkaline earth metal benzoate particles according to any one of claims 1-4 are used.

9. Use of alkali or alkaline earth metal benzoate as a carrier.

10. Use of alkali or alkaline earth metal benzoate particles according to any one of claims 1-4 in a capsule or a sachet.
